(19) Europäisches Patentamt
European Patent Office
Office européen des brevets

(11) EP 0 475 189 B1

(12) **EUROPÄISCHE PATENTSCHRIFT**

(45) Veröffentlichungstag und Bekanntmachung des Hinweises auf die Patenterteilung:
**19.06.1996 Patentblatt 1996/25**

(51) Int. Cl.$^6$: **A61L 27/00**

(21) Anmeldenummer: **91114373.3**

(22) Anmeldetag: **27.08.1991**

(54) **Implantatmaterial**

Implant material

Matériau d'implantation

(84) Benannte Vertragsstaaten:
**AT BE CH DE DK ES FR GB GR IT LI LU NL SE**

(30) Priorität: **10.09.1990 DE 4028683**

(43) Veröffentlichungstag der Anmeldung:
**18.03.1992 Patentblatt 1992/12**

(73) Patentinhaber: **MERCK PATENT GmbH D-64271 Darmstadt (DE)**

(72) Erfinder:
- **Bauer, Hans-Jörg**
  **W-6107 Reinheim (DE)**
- **Katzenmeier, Bianca**
  **W-6108 Weiterstadt (DE)**
- **Kuntz, Matthias, Dr.**
  **W-6100 Mühltal-Traisa (DE)**

(56) Entgegenhaltungen:
**EP-A- 0 141 004     EP-A- 0 268 463**
**EP-A- 0 303 941     EP-A- 0 324 425**
**EP-A- 0 382 047**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Implantatmaterial auf Basis von Knochenkeramik.

An leistungsfähiges medizinisches Implantatmaterial für den Knochenersatz wird die Forderung gestellt, daß es eine hohe mechanische Stabilität aufweist. Implantatwerkstoffe auf mineralischer Basis gewährleisten meist nur dann eine hohe mechanische Stabilität, wenn sie als Keramiken, d.h. also in Form von bei ausreichend hohen Temperaturen gesinterten Materialien bzw. Werkstücken eingesetzt werden.

Für den Einheilungsprozeß werden solche Implantatmaterialien als besonders günstig angesehen, die eine hohe Bioaktivität aufweisen, nämlich dahingehend, daß sie vom Organismus angenommen und in ihn integriert werden. Im Falle von Knochenersatzmaterial bedeutet dies, daß es bald mit körpereigenem Gewebe, insbesondere mit dem Knochen, fest und dauerhaft verwachsen soll.

Knochenersatzmaterialien auf Basis von Calciumphosphat-Keramiken gelten aufgrund ihrer chemischen Verwandtschaft mit der Mineralphase natürlicher Knochen als bioaktiv. Natürlicher Knochen besteht in seiner Mineralphase überwiegend aus Hydroxylapatit, einem Calciumphosphat der Summenformel $Ca_5(PO_4)_3OH$.

Hydroxylapatit synthetischen oder organischen Ursprungs, etwa aus natürlichem Knochenmaterial, ist daher ein häufig verwendeter Rohstoff zur Herstellung von Implantaten für den Knochenersatz. Hydroxylapatit-Keramik ist im Organismus im wesentlichen nicht resorbierbar. Das heißt, das körperfremde Material bleibt über lange Zeit praktisch unverändert erhalten und die Integration in den Organismus erfolgt im wesentlichen durch Verwachsen mit vorhandenem Knochen und Einwachsen im umgebenden Gewebe.

Die gegenwärtig verfügbaren keramischen Implantatmaterialien auf Basis von Calciumphosphat gliedern sich in zwei grundsätzliche Gruppen.

Die erste Gruppe verwendet synthetisch hergestellte Calciumphosphate, die zu kompakten oder porösen Körpern geformt und anschließend zur Keramik gesintert werden. Der Vorteil dieser Materialien liegt naturgemäß in der Tatsache, daß der synthetische Aufbau problemlos gezielte chemische Zusammensetzungen mit großer Exaktheit und Reproduzierbarkeit ermöglicht. Die Standardisierbarkeit der Zusammensetzung ist für medizinische Anwendungen unerläßlich.

Die Festigkeit der Verwachsung von kompakter Calciumphosphat-Keramik mit vorhandenem Knochen ist erfahrungsgemäß überwiegend nicht befriedigend. Ein günstigeres Einwachsverhalten zeigen poröse Calciumphosphat-Keramiken.

Ein entscheidender Nachteil synthetischer Materialien ist, daß poröse Körper nur sehr schwierig und mit hohem Aufwand hergestellt werden können. Es ist bis heute noch nicht möglich, mit synthetischen Materialien Formkörper mit der für natürliche Knochen charakteristischen Porosität, insbesondere etwa der offenen Porosität von Spongiosaknochen nachzustellen. Es hat sich aber herausgestellt, daß gerade diese knochentypische Porosität für eine schnelle, feste und dauerhafte Verbindung von Implantat mit dem körpereigenen Knochen essentiell ist.

Die zweite Gruppe basiert auf natürlichem Knochen, der durch verschiedene Behandlungen mineralisiert und in ein keramisches System überführt wird, wobei die Struktur des Knochens möglichst erhalten bleiben soll. Den Verfahren gemeinsam ist die Entfernung der organischen Knochenbestandteile und die anschließende Verfestigung zur Keramik durch Sinterung bei entsprechenden Temperaturen. Die Entfernung der organischen Anteile erfolgt durch chemische Lösungsvorgänge oder durch pyrolytische Verfahren. Knochenkeramikimplantate zeigen aufgrund ihrer ausgezeichneten Übereinstimmung mit dem Porensystem natürlichen Knochens erhebliche biologische Vorteile beim Einwachsverhalten und der Heilung in Organismus.

Nachteilig an Knochenkeramik ist, daß das als Ausgangsmaterial eingesetzte Naturprodukt Knochen in der chemischen Zusammensetzung seiner Mineralphase erheblichen naturbedingten und nicht kontrollierbaren Schwankungen unterworfen ist.

Zwar besteht die Mineralphase von Knochen überwiegend aus Hydroxylapatit, sie enthält aber auch von Tierart zu Tierart unterschiedliche, ja sogar von Individuum zu Individuum schwankende Anteile an anderen Calciumphosphatphasen, Spurenelementen und insbesondere an Calciumcarbonat. So haben beispielsweise Pferdeknochen in der Regel einen deutlich höheren Anteil an Calciumcarbonat als Knochen vom Rind. Der Calciumcarbonatanteil von Knochen kann durchaus im Bereich zwischen 1 und 25 Gew.% variieren.

Es ist daher praktisch nicht möglich, beim Ausgangsmaterial eine gleichbleibende einheitliche chemische Zusammensetzung vorauszusetzen, die erforderlich wäre, um ein standardisiertes Knochenkeramikprodukt zu erhalten. Derartige Schwankungen in der Zusammensetzung haben durchaus deutlich feststellbare Einflüsse auf die biologische Aktivität des Knochenkeramikimplantats. So wird im Organismus der pH-Wert des das Implantat umgebenden Milieus durch den Gehalt an durch das Brennen aus Calciumcarbonat entstandenen Calciumoxid beeinflußt, was sich klinisch in unterschiedlichen Einwachs- und Knochenneubildungsraten manifestiert.

Als ein besonderes Problem hat sich weiterhin herausgestellt, daß Knochenkeramik zu unkontrollierter Instabilität neigt. Sowohl bei der Lagerung als auch im Organismus können bei Knochenkeramikimplantaten nach unterschiedlicher und nicht vorhersehbarer Zeit Festigkeitsverluste auftreten, die bis hin zum Zerfall führen.

Der vorliegenden Erfindung lag daher die Aufgabenstellung zugrunde, ein Implantatmaterial auf Basis von Knochenkeramik zu entwickeln, das die Nachteile der wechselnden, den natürlichen Schwankungen unterlegenen chemischen Zusammensetzung und der unvorhersehbaren Instabilität nicht aufweist.

Es wurde nun gefunden, daß eine Knochenkeramik erhalten werden kann, in der die poröse Feinstruktur des Knochens im wesentlichen unverändert vorliegt und die unabhängig von der chemischen Zusammensetzung der Mineralphase des ursprünglichen Knochens zu mehr als 99 % aus Hydroxylapatit besteht, wenn man von allen organischen Bestandteilen befreites Knochenmaterial einer Behandlung mit einer wäßrigen Lösung einer organischen Säure aus der Gruppe der aliphatischen $C_{1-5}$-Monocarbonsäuren sowie Malonsäure, Tartronsäure, Bernsteinsäure, Äpfelsäure, Weinsäure und Zitronensäure unterzieht und anschließend zur Keramik sintert. Auf diesem Wege ist somit eine Knochenkeramik von gleichbleibend definierter Zusammensetzung erhältlich.

Überraschenderweise zeigt ein derartiges Material nicht die Instabilität herkömmlicher Knochenkeramik.

Gegenstand der Erfindung ist somit ein Implantatmaterial auf Basis von natürlichem Knochen, bei dem es sich um ein gesintertes Keramikmaterial handelt, in der die poröse Feinstruktur des Knochens im wesentlichen unverändert vorliegt und das zu mehr als 99 % aus Hydroxylapatit besteht.

Gegenstand der Erfindung ist auch ein Verfahren zur Herstellung von einem derartigen Knochenkeramik-Implantatmaterial, bei dem man von allen organischen Anteilen befreites Knochenmaterial einer Behandlung mit einer wäßrigen Lösung einer organischen Säure aus der Gruppe der aliphatischen $C_{1-5}$-Monocarbonsäuren sowie Malonsäure, Tartronsäure, Bernsteinsäure, Äpfelsäure, Weinsäure und Zitronensäure unterzieht und anschließend bei Temperaturen zwischen 900 und 1400 °C sintert.

Eigene Untersuchungen zur Instabilität von üblichen Knochenkeramiken haben ergeben, daß der Zutritt von Feuchtigkeit ein auslösendes Moment für die Instabilität zu sein scheint. Für den Zeitraum der Lagerung ließe sich dieses Problem zwar durch feuchtigkeitsdichte Verpackung der Keramik lösen, nicht jedoch nach der chirurgischen Applikation, nach der das Knochenkeramikimplantat naturgemäß dem wäßrigen Medium des Organismus ausgesetzt ist. Als ursächlich für die Instabilität wurden die wechselnden Gehalte von Calciumcarbonat in dem ursprünglichen Knochenmaterial identifiziert, wobei folgender Mechanismus als wahrscheinlich angesehen werden kann: Während der thermischen Behandlungsschritte bei der Umwandlung des Knochens zur Keramik wird in der Mineralphase vorhandenes Calciumcarbonat in Calciumoxid umgewandelt. In der gesinterten Keramik liegen somit dann in stöchiometrisch equivalentem Anteil Calciumoxidphasen vor. Bei Zutritt von Wasser, etwa in Form von Luftfeuchtigkeit bei ungeschützter Lagerung oder im Organismus nach der Implantation, tritt eine sukzessive Umwandlung von Calciumoxid in Calciumhydroxid gemäß der Formelgleichung

$$CaO + H_2O \rightarrow Ca(OH)_2$$

ein. Mit dieser Umwandlung geht in den Calciumoxidphasen eine Volumenzunahme einher, die rechnerisch bei ca. 97 % liegt. Hierdurch werden in dem Keramikwerkstoff sukzessive Spannungen und Haarrisse erzeugt, die in einer Quellung und Zerrüttung bis hin zum Zerfall der Keramik in Einzelpartikel münden können.

In dem erfindungsgemäßen Verfahren werden vor der Sinterung zur Keramik aus dem mineralisierten Knochen alle vorhandenen Anteile von Calciumcarbonat und gegebenenfalls Calciumoxid sowie, soweit vorhanden, untergeordnete Anteile sonstiger löslicher Inhaltstoffe gelöst. Die Säurebehandlung wird zweckmäßigerweise mit wäßrigen Lösungen schwacher organischer Säuren durchgeführt. In erster Linie kommen hierfür aliphatische $C_{1-5}$-Monocarbonsäuren, aliphatische di- und tri-Carbonsäuren sowie deren Hydroxy-Derivate, jeweils mit insgesamt bis zu 6 C-Atomen, in Betracht. Beispiele derartiger Säuren sind Ameisensäure, Essigsäure, Propionsäure, Buttersäure, Valeriansäure, Malonsäure, Tartronsäure, Bernsteinsäure, Äpfelsäure, Weinsäure und Zitronensäure.

Als besonders geeignet, da leicht verfügbar, wohlfeil und äußerst problemlos zu verarbeiten, hat sich Zitronensäure erwiesen.

Die zu wählende Einsatzkonzentration der Säurelösungen hängt ab von Art und Säurestärke der Säure, der vorgesehenen Behandlungsdauer, der Temperatur und der Art, wie das Knochenmaterial vorbehandelt wurde. In der Regel liegt die Einsatzkonzentration zwischen 1 und 30 Gew.%, vorzugsweise zwischen 5 und 10 Gew.%. Die Temperatur der Säurelösung kann frei zwischen 20 °C und Siedetemperatur eingestellt werden. Je nach Menge des zu behandelnden Knochenmaterials, nach Größe der Stücke sowie der vorgenannten Parameter ist ein Behandlungszeitraum zwischen 2 Minuten und 24 Stunden ausreichend. Anhand der vorstehenden Rahmenbedingungen kann der Fachmann ohne weiteres durch wenige Routineversuche die jeweils geeignetsten Verfahrensparameter ermitteln.

Alles in allem sind die gegebenen Parameter für die Säurebehandlung lediglich als grobe Richtschnur zu betrachten, deren Über- bzw. Unterschreitung in der Regel keinen sonstigen nachteiligen Effekt haben, sofern gewährleistet ist, daß jegliches Calciumoxid entfernt ist.

Zunächst ist der als Ausgangsmaterial dienende Knochen vollständig zu mineralisieren, d.h. von allen organischen Anteilen zu befreien. Dies kann mit der mechanischen Beseitigung von etwa noch anhaftenden Weichteilen beginnen, beinhaltet aber immer die Entfernung der im wesentlichen aus Collagen bestehenden organischen Phase des Knochens. Hierfür sind verschiedene Verfahrensweisen bekannt. Dies kann beispielsweise durch chemische Lösungs- und Extraktionsprozesse geschehen, etwa durch Auskochen und/oder Behandlung mit fett- bzw. eiweißlösenden Lösungsmitteln und/oder Behandlung mit Wasserstoffperoxid. Als besonders einfach und effektiv haben sich aber pyrolytische Verfahren erwiesen. Hierbei wird durch Hitzeeinwirkung der organische Anteil des Knochens zersetzt und der resultierende Kohlenstoff im Sauerstoffüberschuß ausgebrannt. Für die Knochenpyrolyse sind Temperaturen zwischen 500 und 1000 °C, vornehmlich zwischen 600 und 800 °C üblich. Zur Mineralisierung des Knochenmaterials können auch chemische Lösungsvorgänge und Pyrolyseverfahren kombiniert werden. Bei all diesen Verfahren ist darauf zu achten, daß die poröse Feinstruktur des natürlichen Knochens so weit als möglich erhalten bleibt. Für das erfindungsgemäße Knochenkeramik-Implantatmaterial ist es essentiell, daß diese Struktur im wesentlichen unverändert bleibt, so daß die genannten Maßnahmen möglichst schonend durchgeführt werden sollen. Für die Pyrolyse kann daher nach einem Verfahren gemäß DE-PS 37 27 606 vorgegangen werden, bei dem eine spezielle Führung von Temperatur und reduktivem bzw. oxidativem Charakter der Atmosphäre eine extrem schonende Pyrolyse ermöglicht.

Es hat sich als vorteilhaft erwiesen, das Knochenmaterial vor der Säurebehandlung einer Vorsinterung zu unterziehen. Hierdurch wird das Strukturgefüge in der Mineralphase des Knochens verfestigt, so daß die Belastungen der bei der Säurebehandlung auftretenden $CO_2$-Entwicklung unbeschadet überstanden werden. Besonders wichtig ist dies bei Spongiosaknochen, dessen hochporöse grazile Struktur naturgemäß besonders empfindlich ist.

Die Vorsinterung erfolgt zweckmäßigerweise bei Temperaturen zwischen 600 und 1000 °C, vorzugsweise um 900 °C.

Im Falle der Notwendigkeit einer Vorsinterung ist es zweckmäßig, diesen Schritt gleich an den Pyrolyseschritt anzuschließen und somit beide Schritte in einem kombinierten Verfahrensgang durchzuführen.

Für die Säurebehandlung genügt es, das so vorbehandelte Knochenmaterial in ein Bad der Säurelösung zu verbringen und dort für eine als ausreichend und zweckmäßig erscheinende Zeit und bei entsprechender Temperatur zu lagern. Eine Umwälzung der Säurelösung kann der Herauslösung der löslichen Bestandteile förderlich sein.

Nach abgeschlossener Säurebehandlung wird das Knochenmaterial säurefrei gewaschen, zweckmäßigerweise mit demineralisiertem Wasser.

Danach erfolgt dann die Endsinterung zur Keramik nach üblichem Verfahren und in hierfür gebräuchlichen Vorrichtungen. Sintertemperaturen zwischen 900 und 1400 °C sind hierfür ausreichend. Typischerweise wird das Material mit ca. 100 °C/h bis zur Endtemperatur, z.B. 1250 °C, aufgeheizt, auf dieser Temperatur ca. 3 bis 5 h gehalten und dann frei abgekühlt.

Die so erhaltene Knochenkeramik besteht chemisch zu mehr als 99 % aus Hydroxylapatit, was sich röntgenographisch, auf Grund der Genauigkeit dieser Untersuchungsmethode, eindeutig nachweisen läßt. Insbesondere enthält sie keine Anteile mehr an Calciumoxid, das die Ursache für die Instabilität herkömmlicher Knochenkeramik ist.

Das erfindungsgemäße Knochenkeramik-Implantatmaterial vereint in bislang noch nicht gekannter Weise das vorteilhafte Einwachsverhalten aufgrund der im wesentlichen unverändert vorliegenden porösen Struktur des zugrundeliegenden Knochens mit der für derartige Materialien bisher nie erreichten gleichbleibend definierten chemischen Zusammensetzung, nämlich der von Hydroxylapatit von zu mehr als 99 % Reinheit. Insbesondere tritt bei dem erfindungsgemäßen Material das Problem unkalkulierbarer Instabilität nicht mehr auf.

Beispiel 1

Rohe, von Weichteilen befreite Rinderknochen wurden mittels Säge zerkleinert und 3 mal ca. 1 h lang mit Wasser ausgekocht. Die Knochenstücke wurden dann unter Stickstoffatmosphäre mit 50 °C/h auf 450 °C erhitzt. Während einer anschließenden Aufheizperiode mit 25 °C/h auf 750 °C wurde die Atmosphäre linear auf Luftsauerstoff umgestellt und noch 8 h bei dieser Temperatur gehalten. Im Anschluß wurde mit 50 °C/h auf eine Vorsintertemperatur von 900 °C aufgeheizt. Nach dem Abkühlen wurden die Stücke für 3 h in eine umgewälzte 5 Gew.%ige Zitronensäurelösung verbracht.

Nach der Entnahme aus dem Säurebad wurde mit demineralisiertem Wasser neutral gewaschen. Zur Endsinterung wurden die Stücke mit 100 °C/h auf 1250 °C erhitzt, 3 h auf dieser Temperatur gehalten und dann frei abgekühlt.

Die erhaltenen Knochenkeramikstücke zeigen die unveränderte poröse Struktur des ursprünglichen Knochens.
Röntgenographisch besteht die Keramik zu mehr als 99 % zus Hydroxylapatit.

Beispiel 2

Bei gleicher Behandlung von Pferdeknochen wurde eine Knochenkeramik erhalten, die ebenfalls zu mehr als 99 % aus Hydroxylapatit besteht.

Beispiel 3 (Vergleich)

Pferdeknochen wurden der gleichen Behandlung unterzogen wie in Beispiel 1, jedoch wurde die Säurebehandlung weggelassen. Die erhaltene Knochenkeramik enthält röntgenographisch ca. 10 % Calciumoxid.

**Patentansprüche**

1. Implantatmaterial auf Basis von natürlichem Knochen, dadurch gekennzeichnet, daß es sich um ein gesintertes Keramikmaterial handelt, in der die poröse Feinstruktur des Knochens im wesentlichen unverändert vorliegt, und das röntgenographisch zu mehr als 99 % aus Hydroxylapatit besteht.

2. Verfahren zur Herstellung von Implantatmaterial nach Anspruch 1, dadurch gekennzeichnet, daß man von allen organischen Anteilen befreites Knochenmaterial einer Behandlung mit einer wäßrigen Lösung einer organischen Säure aus der Gruppe der aliphatischen $C_{1-5}$-Monocarbonsäuren sowie Malonsäure, Tartronsäure, Bernsteinsäure, Äpfelsäure, Weinsäure und Zitronensäure unterzieht und anschließend bei Temperaturen zwischen 900 und 1400 °C sintert.

3. Verfahren nach Anspruch 2, dadurch gekennzeichnet, daß man die Säurebehandlung mit einer wäßrigen Lösung, die 1 bis 30 Gew.% der organischen Säure enthält, durchführt.

4. Verfahren nach den Ansprüchen 2 oder 3, dadurch gekennzeichnet, daß man die Säurebehandlung für einen Zeitraum von 2 Minuten bis 24 Stunden bei einer Temperatur zwischen 20 °C und Siedetemperatur durchführt.

5. Verfahren nach den Ansprüchen 2 bis 4, dadurch gekennzeichnet, daß man die Säurebehandlung mit einer wäßrigen Lösung von Zitronensäure durchführt.

6. Verfahren nach den Ansprüchen 2 bis 5, dadurch gekennzeichnet, daß man das Knochenmaterial vor der Säurebehandlung einer Vorsinterung bei Temperaturen zwischen 600 und 1000 °C unterzieht.

**Claims**

1. Implant material based on natural bone, characterised in that it is a sintered ceramic material in which the porous fine structure of the bone is present in essentially unchanged form, and which consists radio-graphically to more than 99% of hydroxyapatite.

2. Process for the production of implant material according to Claim 1, characterised in that bone material freed from all organic constituents is subjected to treatment with an aqueous solution of an organic acid from the group consisting of the aliphatic $C_{1-5}$-monocarboxylic acids and malonic acid, tartronic acid, succinic acid, malic acid, tartaric acid and citric acid and is then sintered at temperatures between 900 and 1400°C.

3. Process according to Claim 2, characterised in that the acid treatment is carried out using an aqueous solution which contains 1 to 30% by weight of the organic acid.

4. Process according to Claim 2 or 3, characterised in that the acid treatment is carried out for a period of 2 minutes to 24 hours at a temperature between 20°C and boiling temperature.

5. Process according to Claims 2 to 4, characterised in that the acid treatment is carried out using an aqueous solution of citric acid.

6. Process according to Claims 2 to 5, characterised in that the bone material is subjected to presintering at temperatures between 600 and 1000°C before the acid treatment.

**Revendications**

1. Matériau d'implant à base d'os naturel, caractérisé en ce qu'il s'agit d'une matière céramique frittée dans laquelle la structure fine poreuse de l'os se présente essentiellement de façon inchangée, et qui se compose selon l'analyse aux rayons X à plus de 99% d'hydroxylapatite.

2. Procédé de fabrication d'un matériau d'implant selon la revendication 1, caractérisé en ce qu'on soumet la matière osseuse débarrassée de toutes les fractions organiques à un traitement avec une solution aqueuse d'un acide organique du groupe constitué par les acides monocarboxyliques en $C_1$ à $C_5$ aliphatiques ainsi que l'acide malonique, l'acide tartronique, l'acide succinique, l'acide malique, l'acide tartrique et l'acide citrique, puis qu'on la fritte à des températures comprises entre 900 et 1400°C.

3. Procédé selon la revendication 2, caractérisé en ce qu'on effectue le traitement acide avec une solution aqueuse qui contient de 1 à 30% en poids de l'acide organique en question.

4. Procédé selon les revendications 2 ou 3, caractérisé en ce qu'on effectue le traitement acide pendant un intervalle de 2 minutes à 24 heures à une température comprise entre 20°C et la température d'ébullition.

5. Procédé selon les revendications 2 à 4, caractérisé en ce qu'on effectue le traitement acide avec une solution aqueuse d'acide citrique.

6. Procédé selon les revendications 2 à 5, caractérisé en ce qu'on soumet la matière osseuse avant le traitement acide à un pré-frittage à des températures comprises entre 600 et 1000°C.